# EUROPEAN PATENT SPECIFICATION

(11) **EP 2 569 487 B1**
(45) Date of publication and mention of the grant of the patent: **23.11.2016**
(21) Application number: 11731456.7
(22) Date of filing: 13.05.2011
(51) Int. Cl.: E03D 9/03

(54) **DISPENSING DEVICE AND METHOD OF MANUFACTURE**
WC-ABGABEVORRICHTUNG UND HERSTELLUNGSVERFAHREN
DISTRIBUTEUR DE TOILETTE ET PROCEDE

(30) Priority: 14.05.2010 GB 201008135
(43) Date of publication of application: 20.03.2013
(73) Proprietor: Jeyes Group Limited, Norfolk IP24 1HF (GB)
(72) Inventor: WILSON, Brian, Norfk IP22 2BB (GB); STEVENSON, Matthew, Norfk IP25 7DJ (GB); PAYNE, Trevor, Norfk IP24 3JZ (GB)
(74) Representative: Martin, Philip John
(86) International application number: PCT/GB2011/000744
(87) International publication number: WO 2011/141720

(56) References cited:
- AT-B- 293 965
- US-A- 3 088 126
- US-A- 3 217 338
- US-A- 4 096 593

## Description

The present invention relates to improvements to a dispensing device and in particular to a device used to deliver a treatment composition to a sanitary appliance, such as a toilet.

The treatment composition contains one or more chemical constituents e.g., colouring agents, cleaning agents, disinfecting agents, anti-lime scale agents in the form of a block. The treatment composition is formed by water contacting the block of the device coming into contact with the one or more chemical constituents; the block provides for the long term release of the one or more active agents during sequential contacts with water contacting the block of the dispensing device.

The first treatment compositions such as paradichlorobenzene were commonly added to the sanitary appliance as a loose block that dissolved over time. Toilet blocks for use as loose blocks are still common today although they are more likely to be Naphthelene based products.

Typically, lavatory cleansing blocks are immersed in the water cistern of a lavatory (an in-tank or in-cistern block), or held in a cage under the rim of the lavatory bowl (a rim block), or sometimes in a cage on the top of the cistern, in Japan for example, (an on-tank block). The block dissolves slowly, releasing active ingredients into the lavatory bowl.

Generally such blocks may contain as active ingredients one or more of:
i) a surface active agent to provide a cleansing effect, and also to provide foam as an indicator to the user;
ii) a bleach component;
iii) a colourant primarily as an indicator to the user;
iv) a perfume;
v) a germicide;
vi) limescale controller.

It will be appreciated that the extent to which the block provides a cleansing action pre se depends on the active ingredients used and the quantity dosed into the lavatory bowl during a flush cycle.

The block will also contain processing aids to assist in manufacture of the block by extrusion, tableting, etc, fillers and also solubility controllers to control the lifetime of the block. The in use life of the block is governed in particular by the amount of material in the block and the rate at which the block dissolves in the flush water and this depends primarily on the composition of the block.

Also, the block life will depend in part on the hardness of the flush water, the ambient temperature, the frequency of flushing and even the toilet design. These factors are well known in the art and the block composition can be tested against a range of conditions when determining a suitable composition for a particular market.

The quantity of active ingredients delivered into the toilet bowl during each flush cycle of the toilet will in turn affect the actual and perceived performance of the block: for example the action of a surfactant or bleach to cleanse the toilet bowl, and the appearance of foam or dye to the user, or perception of a fragrance.

Devices for dispensing treatment compositions are well known for use with household, sanitary appliances and in particular modern flush toilets. Two well known types of device are intended to be used as "in the bowl" (ITB) or "in the cistern" (ITC) devices in order to provide a colouring and/or cleaning and/or fragrancing and/or sanitizing effect to such sanitary devices, particularly toilet bowls. One common approach known in the art is to provide a device which is at least immersed within the cistern or tank of a toilet, which may be either placed wholly within the interior of the toilet such as by placement at the bottom of a toilet tank so that the entire device is wholly immersed in water when the tank is full, or is at least partially immersed within the water present in a toilet tank, such as wherein such a device is suspended from a part of the toilet tank, such as a lip or rim of the tank. Such are generally referred to as ITC devices.

A further common approach known to the art is to provide a device which is suspended from the rim of the toilet bowl and which is placed at or near the interior sidewall of the toilet bowl. Such are generally referred to as ITB devices. Such a device is designed to typically dispense a treatment composition to the interior of a toilet when a gel or block composition is contacted with flushing water, or alternately, dispensing a fragrancing composition to the toilet bowl which is intended to counteract or mask malodours. Typically, in today's market such devices include a hanger portion which is used to suspend a cage portion from the rim of the toilet bowl, such that the cage portion is positioned within the path of flowing water which is dispensed with each flush operation of the toilet. The cage portion typically comprises a plurality of holes or apertures which permit for the flush water to both enter and to exit the cage portion of the device. Typically a solid block composition or a gel composition is present within the cage. The solid block composition and/or gel composition typically comprises one or more cleaning constituents, e.g., one or more surfactants which provide a good cleaning and/or foaming benefit. Often the solid block composition and/or gel composition comprises a fragrance constituent as well which is provided to provide some degree of malodour suppression. For most such devices, the use of a cage is considered desirable especially for use with gel compositions, as gels are not self supporting and would not be useful without the physical supporting structure provided by the cage. With regard to solid block compositions, such compositions are notoriously prone to weakening and softening over time and most are known to swell or sag over their lifetime, particularly when approaching the end of their useful service life. The cage acts then as a porous receptacle and support for said blocks that would otherwise prematurely soften or disintegrate and fall into the toilet bowl and be flushed away before their composition is substantially consumed.

While the use of a cage is beneficial for use with certain block compositions, the use of a cage also has disadvantages. The use of a cage requires increased material costs, and additional manufacturing steps. Furthermore, such ITB devices are often single use type devices, once the gel or block composition is consumed or otherwise exhausted, the consumer discards the entire ITB device which is wasteful and contributes to the problems associated with proper garbage disposal. With regard to costs, in most conventional rim suspended lavatory devices comprising a hanger portion and a cage portion, the bulk of the material is typically used to form the cage. As such cages are typically fabricated from a synthetic polymer, such requires specific moulding operations in order to form the rim suspended lavatory device, and to fill the cage with the solid block composition and/or gel composition prior to use and or sale.

Known to the art are rim suspended lavatory devices which are lavatory blocks of paradichlorobenzene which provide no cleaning benefit, but provide only a fragrancing benefit. Such blocks typically erode per sublimation of the paradichlorobenzene and/or by contact with flush water. Such rim suspended lavatory blocks of paradichlorobenzene are typically packaged as a solid block or cake having extending from one side a loop of bendable wire. A portion of the bendable wire is embedded within the paradichlorobenzene block. The consumer is required to form the wire into a hanger appropriate to the particular geometry of their toilet so that the paradichlorobenzene block is positioned with the interior of the toilet bowl.

EP-A-1891197 discloses a process for the manufacture of a lavatory dispensing device useful for the delivery of at least one treatment composition, preferably a cleaning composition and or a sanitizing composition to a sanitary appliance, preferably a toilet bowl, comprising, providing a composition to an extruder, forming an extrudate from the composition, inserting a part of a hanger into said extrudate, compressing the extrudate to encase or enrobe said part of a hanger thereby forming said lavatory device.

WO-A-2007107750 and WO-A-2007107755 described certain cageless lavatory dispensing devices which include solid block compositions formed by extruding a mass(es) of a lavatory treatment composition, and thereafter stamping or compressing the extrudate in a die or pair of dies in order to densify the solid block compositions and to simultaneously attach them to a hanger. While said devices function adequately, it has been observed that the compositions may be inadequately stable for long periods of storage at high temperatures wherein it has been observed that the stamped solid block compositions may be deformed or slump when packaged. It has also been observed that rough handling of the said devices when packaged in blister-type packaging may suffer deformation at contact points with the blister-type packaging which is unattractive from a consumer standpoint. Such deformation is due to the fact that the device is inserted within a cavity of the blister, but is otherwise free to move within the cavity. Depending upon the configuration of the cavity and that of the stamped solid block compositions there may be one or more contact points, such as at edges of the stamped solid block compositions, the device moving within the confines of the cavity may be deformed, or smear at such contact points which provides for an unattractive appearance, and depending upon the composition of the block and that of the ambient temperature of the environment wherein the device is removed from the blister-type package, may also provide for undesirable sticking or adhesion between the blister of the package and the stamped solid block compositions.

Thus, while certain known-art dispensing devices provide beneficial treatment effects, there is nonetheless a real and continuing need in the art to provide still further improved devices which can provide to a sanitary appliance a useful treatment benefit, preferably a useful cleaning benefit, and which overcome one or more of the shortcomings of prior art devices.

WO-A-2010001091 discloses a process for the manufacture of a cageless lavatory dispensing device comprising a hanger having a part thereof adapted to be suspended from a part of a sanitary appliance, particularly from a part a toilet cistern or toilet tank, and a cast solid block comprising one or more chemical constituents, wherein the device is adapted to be suspended within the interior of said cistern or tank. A related application, WO-A-2010001092, discloses a cageless lavatory dispensing device comprising a hanger having a hook end adapted to be suspended from a part of a sanitary appliance, preferably the rim of a toilet bowl, and a cast solid block comprising at least one chemical agent adapted to be suspended within the interior of the sanitary appliance.

The above noted documents relate to modern equivalents of well known cageless dispensing devices such as those described in US-A-3088126, US-A-3217338, US-A-3290699, US-A-3604021, US-A-3668717, US-A-5987655, DE-A-8906140 and EP-A-1418225.

US-A-3604021 shows that the basic concept of a cageless block for an ITB has been well known since the 1960's.

DE-A-8906140 (Also CH675140) discloses a product where the block is enclosed in a bag attached to the hook.

US-A-3668717 discloses an embedded plastic hook having a top plate to prevent compositions disintegrating too quickly. The stem and plate of the hanger are inserted into a hot mix for the bar in a mould whereupon the mix is allowed to solidify around the stem and under the plate. The document suggests that (cast) blocks on hangers were well known before 1970 - i.e. cageless dispensing devices for toilet blocks. DE 1879599 (U) discloses a similar product.

US-A-3947901 suggests that it was common to have cageless paradichlorobenzene blocks hanging in the bowl. The paradichlorobenzene block is pressed or moulded onto the hanger.

GB-A-2368854 discloses a dispenser for releasing a substance into a toilet bowl consisting of an elongate resilient member having a plurality of protrusions which engage and hold the substance. The substance to be released may be a gel, soap or crystalline salt, and may be extruded onto the skeleton.

EP-A-1287108 (WO 01/88078) discloses a cleansing device, including a skeleton for supporting a solid bar. The solid bar is transparent.

Thus there has been an historical move away from 'cageless' ITB devices and also 'cageless' ITC devices. Toilet blocks on hangers, in the absence of a cage, are no longer common as they have not been considered commercially desirable. Furthermore, with the advent of modern compositions including high levels of surfactants and new gel compositions, it has been considered that a cage was useful to prevent a messy appearance in the bowl as some compositions were liable to become mushy. It is not clear that the skilled person was in any doubt that useable compositions including the new materials were possible; however, there was a strong commercial driver to produce caged devices.

### SUMMARY OF THE INVENTION

The present invention provides various improvements to the field of cageless dispensing devices in particular, relating to the hanger design, the method of manufacture and the composition of the toilet block.

In an embodiment of the invention the block is produced by extruding the desired composition and inserting pins on the hanger directly into the block to support the extruded block on the hanger. The invention provides a dispensing device having a sanitary block pinned to the hanger. A portion of the hanger abutting an external surface of the sanitary block through which the finger is pinned.

The invention provides a toilet bowl treatment composition dispensing device, comprising a hanger and a treatment composition block, the hanger comprising, an elongate body portion having a first end and a second end, a hook portion at the first end for suspending the hanger from a rim of a toilet bowl, and a sanitary block supporting portion at the second end, the sanitary block supporting portion having at least one finger projecting from a surface of the supporting portion, wherein the hook portion and the at least one finger project from opposite longitudinal sides of the body portion, and the treatment composition block is retained on the supporting portion by said at least one finger inserted into a surface of said treatment composition block.

The body portion may define a longitudinal axis of the dispensing device, the hook end extending from one end of the body portion and aligned with the longitudinal axis and the supporting portion extends from the opposite end also aligned with the longitudinal axis. The hook portion may be predominantly disposed on one side of the stem portion defining a rear of the device, the opposite side of the stem portion being the front of the device. The supporting portion may include a planar surface for abutting a face of a treatment block.

The dispensing device may comprise a plurality of fingers each extending from the supporting portion in the same direction. There may be two fingers on the supporting portion.

The at least one finger may be located on a part of the support portion having an enlarged cross section.

The at least one finger and the hanger project on opposite longitudinal sides of the body portion.

The at least one finger may be inserted into a surface of the treatment composition block whereby a surface of the supporting portion from which the at least one finger extends abuts the surface of the treatment composition block into which the finger is inserted. The abutting surfaces may be planar.

The block may be cylindrical.

The block may have two opposing faces that have been subjected to a cutting process and the at least one finger is inserted into a surface of the block other than said opposing faces.

The at least one finger may have at least a portion having a surface that is serrated, barbed or roughened to improve grip.

The or each finger may have a length that is at least 20% of the depth of the block and no more than 100%, preferably 50 to 95%, more preferably 60 to 85%, most preferably 70 to 80%.

The treatment composition block may be provided with a channel in the surface into which the pins inserted and the channel conforms to the profile of the supporting portion which overlaps said surface of the block.

The treatment composition block may be an extruded block. The treatment composition block may be a co- extruded block.

The treatment composition block may comprise 20 to 80wt% of at least one surfactant, preferably including an anionic surfactant, and optionally, one or more pigments or dyes, one or more additives acting as life extending or life shortening agents, one or more perfumes, and filler.

The treatment composition block may comprise 20 to 60wt% of at least one first surfactant and 3 to 20% of at least one second surfactant acting as a plasticizer.

The treatment composition block may comprise the equivalent of 20 to 60wt% of AOS (80%), preferably 25 to 40wt%, more preferably 28 to 35wt%.

The treatment composition block may comprise the equivalent 3 to 20wt% of SAS (92%), preferably 3 to 15wt%, more preferably 4 to 12wt%.

The invention also provides a method of manufacturing a toilet bowl treatment composition dispensing device comprising, providing a hanger including, an elongate body portion having a first end and a second end, a hook portion at the first end for suspending the hanger from a rim of said toilet bowl, and a sanitary block supporting portion at the second end, the sanitary block supporting portion having at least one finger for receiving a treatment composition block and projecting from the planar portion; the hook portion and the at least one finger project from opposite longitudinal sides of the body portion; and inserting said at least one finger into a surface of said treatment composition block.

The treatment composition block may be formed by one of, an extrusion process, a tableting process and a hot melt process.

The method may comprise extruding the treatment composition and cutting into blocks. The extrusion process may be a co-extrusion process and the block has at least two distinct portions having different compositions.

Before inserting the at least one finger the block may be subjected to a shaping process in a mould.

The method may include a step of wrapping the block in a water soluble film.

Since the devices of the present invention are assembled by simply pinning the hangers onto the blocks or the blocks onto the hangers, there is no compression of the block onto the hanger and the pinning process has no material effect on the block - there is no densification.

### BRIEF DESCRIPTION OF THE DRAWINGS

Figure 1 is a schematic showing a process according to an embodiment;
Figures 2a and 2b are perspective views showing variations of a device including a toilet block according to an embodiment;
Figures 3a and 3b are perspective views showing the device according to the embodiment of figures 2a and 2b without the toilet block;
Figure 4a and 4b are perspective views of a further solution with and without a toilet block respectively, not in accordance with the invention;
Figure 5a and 5b are rear and side views of a dispensing device according to a further embodiment;
Figures 6a, 6b, 6c, and 6d show examples of basic shapes for a toilet block, wherein figures 6a and 6b are not in accordance with the invention;
Figure 7 shows one example of a shape of a co-extruded toilet block;
Figure 8 shows an example of a shaped and patterned toilet block.

### DESCRIPTION OF THE EMBODIMENTS

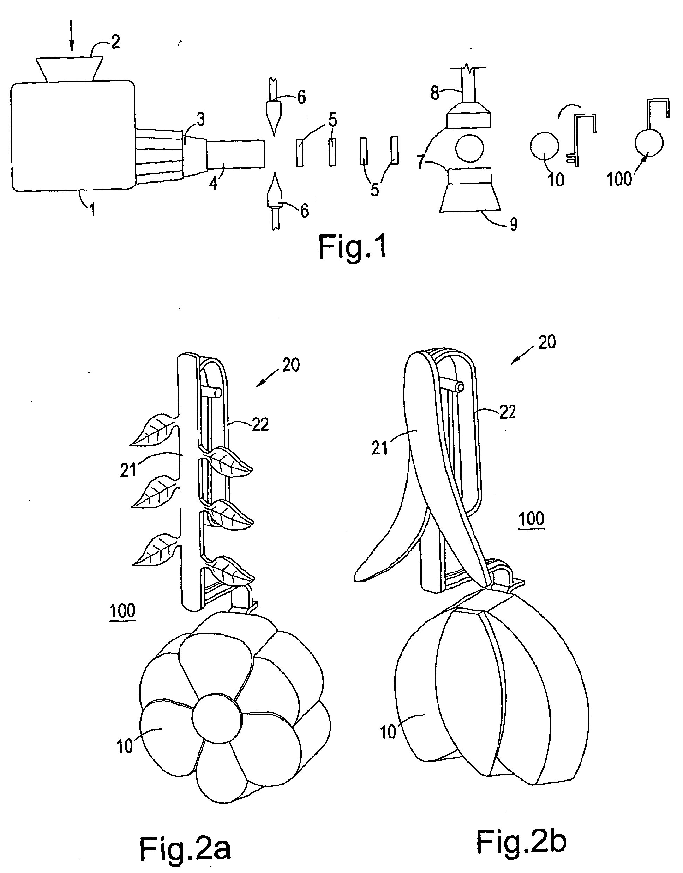

Turning now to the figures, figure 1 shows schematically a process for manufacturing cageless dispensing devices disclosed herein.

A premixed toilet block composition, or the constituents required to form a block composition, is provided to the inlet hopper 2 of an extruder 1. The extruder 1 may be a single screw extruder or a multiple screw extruder. Where plural screws are present, the screws may be co-rotating or may be counter-rotating. If not previously mixed or blended prior to introduction into the extruder, the block composition is formed into a generally homogeneous mass and exits the extruder via a suitable die 3 which has an orifice profile of suitable dimensions. Typically the extrudate 4 has a circular cross section and a diameter of about 50 mm. After exiting the die 3, the extrudate 4 is cut into measured lengths or masses and are separated using one or more cutting blades 6 to form a preform 5. Any suitable means may be used to separate the extrudate 4 into preform 5.

The process of the present embodiment as described above is similar or may be the same as that disclosed in WO-A-2007/107755. However, the two processes diverge at this point. In the process of WO-A-2007/107755 the die shapes the extrudate to include a cavity, channel or recess within the extrudate of suitable dimensions to accept at least the plate of a hanger or, alternatively, an additional process step is performed, for example a channel-cutting step using a channel cutting means such as a rotating winged cutter, saw, plough, blade or other cutting device is applied to or passed through the extrudate or alternatively to the preform in order to split or shape the extrudate or lengths adequately to provide a channel or recess suitably sized to later receive a portion of a hanger. Such a channel or recess extends longitudinally through the extrudate and/or the preform for positioning of the plate of the hanger in a next process step. In an alternative process step, subsequent to- extrusion, the extrudate or preform may be provided with a cavity, such as being partially split using any suitable tool means, such as a suitably dimensioned plunging blade which may be used to partially split a portion of an extrudate or a cut length in order to provide a cavity or slot which is of sufficient width and depth to subsequently accommodate at least a part of a hanger, especially a plate of a hanger. Such a cavity formed by such blade need not extend across the length of the preform nor through the ends thereof. In the preferred process of WO-A-2007/107755, as the extrudate exits the die 114 a channel is provided by extruding through a die which includes a blade or other cutter means which extends into the open cross-section of the die such that as the extrudate exits the die, it is provided with such a channel which partially splits the extrudate into the legs of a "V', which remain attached at the base of each leg. Such a channel may extend across the length of the preform and through the ends thereof. Subsequently, in WO-A-2007/107755, the plate of a hanger is inserted within the interior of the channel or cavity such that the plate is encased within the interior of the preform.

In the process of figure 1, contrary to the process of WO- A-2007/107755, no cavity or channel is provided within the preform. In an embodiment described below a channel may be provided in a major surface of the block so that the hanger is recessed within the block but there is no requirement for a plate on the hanger since in the present invention the block is not affixed to the hanger by embedding a plate within the block and compressing the block as in WO- A-2007/107755. In WO- A-2007/107755, in the die compression step a preform 122 having an inserted hanger is introduced between the opposing dies 130 and the opposing dies 130 are brought together to both form the compressed solid block composition and adhere it to the plate 30, as well as to densify the composition of the compressed solid block by at least 1.5%, preferably at least 2% more than the density of the extrudate from which the compressed solid block 50 is formed. The formed cageless lavatory device 10 is subsequently removed from the die and is ready for use, or alternately may be packaged in a suitable package in order to form a vendible article.

In the process of figure 1 the preform 5 is subsequently next inserted into a mould, which may consist of opposing dies 7. The preform 5 is compressed between the dies 7 by a suitable compression means, such as a ram 8 and anvil 9. Optionally prior to introduction of the preform 5 into a die in the next process step, one or more of the interior surfaces of the mould may be sprayed with a mould release material or other lubricant such as mineral oil or a paraffin oil.

After the preform 5 has been compressed to form the shaped block 10, the shaped block 10 is removed from the mould and optionally allowed to cool. The pins of a hanger (to be described below) are then inserted directly into the surface of the block 10 to form a dispensing device 100 including a treatment composition, in the form of a cageless toilet block. The device maybe packaged to form a vendible article. The term dispensing device may also be used to refer to the hanger before combination with the toilet block.

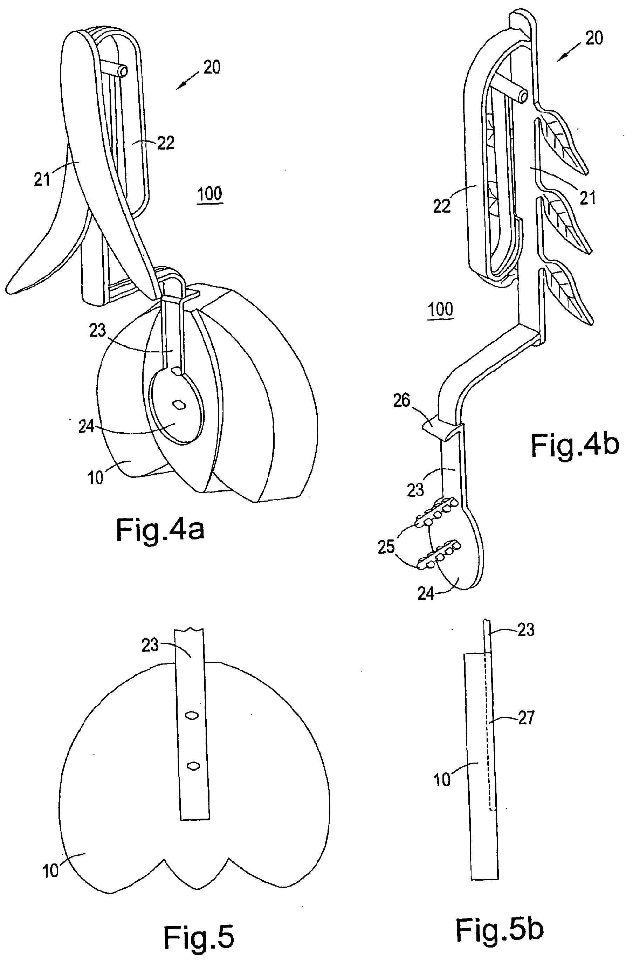

In the most basic form the present invention does not require the moulding step since the product of the extrusion and separating processes may be the finished toilet block which may, for example, be cylindrical; a circular cross section is a known shape for commercial toilet blocks but other shapes are well known. One benefit of the moulding process is that the block can be reshaped to a more attractive three-dimensional design such as those shown in figures 2a, 2b, and 4a. Some compositions could also benefit from the densification of the composition during the moulding/ compressing step.

In an embodiment, the pins of the hanger are inserted directly into the surface of the block after cutting, or if there is a moulding step, after moulding. Depending on the composition of the toilet block, the length of time before insertion of the hanger pins may be varied. Some compositions will allow immediate insertion of the hanger pins and in most cases the hanger pins should be inserted before the block has fully cooled or set so that the composition may receive the pins without damage to the block. If the composition is too hard, when the pins are inserted then internal damage to the block will cause the block to break up to quickly in use. Conversely, for some compositions, if the block has not fully set when the pins are inserted, further setting or curing will allow the block composition to conform to the shape of the pins and to shrink onto the pins to improve the fixing of the block to the hanger.

Whilst many techniques are known for removing moulded products from dies, in an embodiment, one of the dies may have a moveable portion that can be moved either manually or automatically in order to eject the moulded block from the die without breaking or affecting the surface finish.

Figure 1 relates to one possible process route but others are possible, for example:
(1) Extrude to form shaped bars and cut to depth or weight. Hanger simply inserted via a simple jig into block;
(2) Extrude to form shaped bars and cut to depth or weight, compress into mould to reshape or include 3-D pattern, release, then insert hook via simple jig;
(3) Extrude to form shaped bars and cut to depth or weight, tight wrap in water soluble film, compress into mould to reshape or include 3-D pattern, release, then insert hook via simple jig. Optionally, the water soluble film could be pre-split to aid insertion of the pins.
(4) Extrude to form shaped bars and cut to depth or weight, insert pins of hook or hanger in a simple jig, or in a mould, compress into mould to reshape or include 3-D pattern, release, then assembled unit is packed.

The above processes can all be in-line, but equally could be split with the compression/ 3-D reshaping processes etc. could be conducted later.

With regard to option 3 above, water soluble films for toilet blocks are known. Surprisingly, it is found, that such films can assist in the demoulding of the block after shaping. The film could be split in order to ease insertion of the pins and to allow the film to settle in the correct position on the moulded block.

Furthermore, since the disclosed dispensing device is assembled simply by inserting pins of the hanger into a toilet block, the extrusion process is only one possible process; the blocks could also be formed by a tableting process or casting (hot melt) processes as is well known in the art.

Figures 2a and 2b show finished products including a block having a moulded shape produced in the moulding step following extrusion. The blocks 10 are pinned to the hangers 20. The hangers 20 include a decorative stem portion 21 and a hanger or hook portion 22.

The shape of the toilet block resulting from the moulding process will affect the rate of dissolution of the block. Irregular shapes may produce inconsistent dissolution. The shapes shown provide even dissolution and good end of life point.

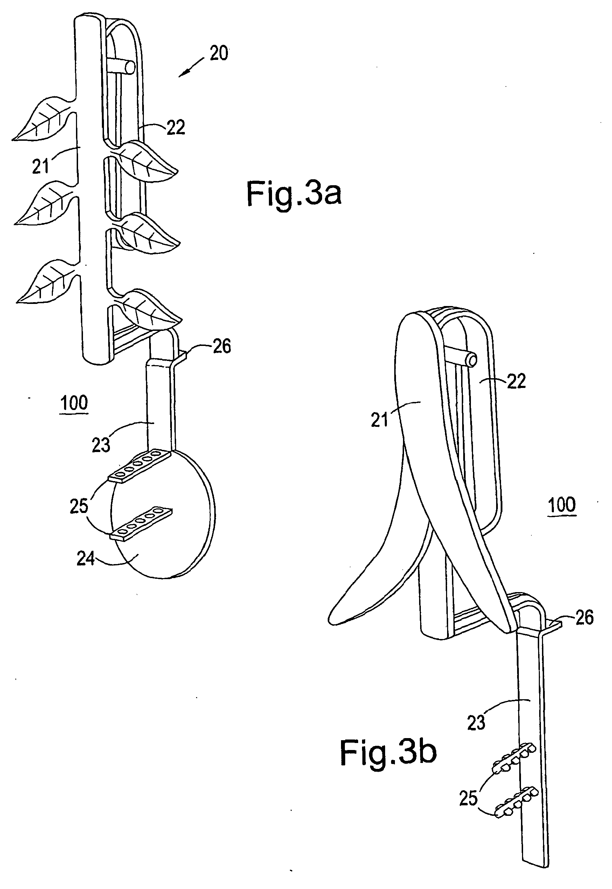

Figure 3a shows a hanger according to an embodiment in accordance with the devices of figures 2a and 2b. The hanger 20 includes block supporting portion 23 which may be a simple extension of the stem 21, but in the present case is arranged offset from the plane of the stem 21 such that when the block 10 is supported on the supporting portion in use the block 10 sits properly against the wall of the toilet bowl or other sanitary appliance. The supporting portion ends in an enlarged portion 24 which abuts the block when the block is affixed and provides additional support. The enlarged portion 24 carries, in the present embodiment, two fingers or pins 25, which are inserted into a surface of the block. In the present embodiment the block has opposing major surfaces one of which is decorative and is intended to face outwards when the device is in position on a toilet bowl rim. The other surface is substantially planar and in abuts a planar surface of the supporting portion 23 and/ or the enlarged portion 24. In the present embodiment the pins 25 are inserted into a non-decorative major surface of the block 10. Of course both sides of the block could be decorative. In the simplest form the block does not need to be decorative and may not have been additionally moulded. The pins 25 could be inserted into any suitable surface of the block.

A variation is shown in Figure 3b which does not include an enlarged portion 24. The supporting portion 23 of figure 3b is straight and again carries two pins 25.

Each of the hangers 20 of figures 3a and 3b include two pins 25. Each pin is serrated or roughened so as to decrease the likelihood of the block slipping off the support. The pins could be barbed or simply roughened. The pins are shown as extending parallel to each other and normal to the plane of the supporting portion 23. However, the pins 25 could also be formed at an angle to the plane of the supporting portion and either diverging or converging in relation to each other. Parallel pins are easier to insert into the block.

The number of pins need not be two and a single pin would suffice to affix the block to the hanger. It is found that two pins improve retention of the block on the hanger, prevent the block rotating relative to the support portion 23, and prevent uneven dissolution and other poor product properties. Other pin configurations are possible, in particular three pins may be preferred. The length of the pins is relevant and a pin length approximately over half the depth of the toilet block (from the surface in which the pins are inserted to the opposing surface) provides a reasonable balance between retaining the block on the support and preventing damage to the block on insertion of the pins. The pins should have a length that is at least 20% of the depth of the block and no more than 100%, preferably 50 to 95%, more preferably 60 to 85%, most preferably 70 to 80%. In a particular example the block is 13mm deep and the pins are 10 mm long.

Each of the hangers 20 of figures 3a and 3b include a stand off portion 26, which may be proportioned so that the block does not rest on the toilet bowl but sits in the flow of flush water. Some compositions will stick to the sanitary ware leaving marks if the device is moved. The portion 26 may be moulded to face in the opposite direction to that shown in figures 2 and 3, and in such configuration the portion 26 acts as a guide for coupling the block to the hanger. See also the embodiment of figure 4 in this regard. In a particular example the block is 12.5mm deep and the pins are 10 mm long.

The hanger is elongate with the stem portion defining a longitudinal axis of the hanger with the hook portion 22 and the supporting portion 23 generally being in line with the axis of the stem portion, that is the axis of each of the portion 21, 22, and 23 are in the same plane.

A benefit of the back pinned toilet dispensers of figures 2 and 3, are that the possibly unsightly enlarged portion 24 is hidden behind the block in use. Since the pins project forward in use, as the block wears away the pins become exposed providing an end of life indication to the user who may discard the dispenser at this point. The back pinned toilet dispensers of figures 3 and 4 also have an overall width (from the front face of the toilet block to the rear part of the hanger) that is more narrow than the front pinned version of figures 4 for example. The back pinned versions therefore pack in a more space efficient manner.

Figures 4a and 4b show an alternative solution of figures 2 and not covered by claim 6. In figure 4, the block 10 is pinned at the front (that is the decorative side of the block and the pins project "backwards" from the supporting portion in the direction of the hook portion 22. In use, therefore, the supporting portion is in front of the block as seen by a user when the device 100 is hooked onto a toilet bowl rim. In this solution the standoff portion 26 does not act on the toilet bowl but instead projects in the same direction as the pins and serves to assist in holding the block on the support. In such a case a single pin may be used since the block is kept from rotating on the pin by the standoff portion 26.

Figure 4a shows a single pin embedded in a block 10 and figure 4b shows a hanger 20 having two pins. The front mounted block device of figures 4a and 4b may provide more consistent positioning of the block relative to the bowl wall.

The rear mounted devices of figures 2 and 3 have the advantage of an improved appearance since the support portion 23 of the hanger does not obscure the shaped toilet block; there is no plastic on the front face of the block and no pins in the centre. On the other hand it is thought that the rear mounted block may have disadvantages in causing design problems in terms of preventing uneven dissolution because of water flow around the support portion. Furthermore, it has been found that the front pinned version of figure 4 retains the block on the hanger more consistently since the hanger applied pressure to the block in use between the enlarged portion 24 and the toilet bowl.

In tests, the front pinned devices have performed better in terms of satisfactory dissolution profiles as compared with the back pinned devices. It is thought that the enlarged portion 24 provides a shield that plays a significant part in how evenly the block dissolves. Alternatively the pressure exerted on the block towards the toilet bowl may allow for more consistent positioning.

The two pin devices also show more consistent and uniform dissolution profiles than one pin devices.

Since the pins of the front pinned device project backwards towards the toilet bowl in use, the pins are hidden from view by the enlarged portion 24 as the block dissolves. The pins project safely away from the user in use.

Figure 5, shows a further embodiment where the shaped block includes a channel 27 in the surface for receiving the support portion 23 of a hanger 20. The depth of the channel 27 is approximately the same as the depth of the support portion 23 as shown in Figure 5b, such that the back of the product is substantially flat. Where the support member abuts the surface of a block 10 not having the channel 27 and protrudes therefrom by the thickness of the support portion, the flush water tends to run down the sides of the support member, which action causes the waterflow concentrations to vary and in particular to be more concentrated around the support portion 23. It is found that placing the support portion 23 in a channel 27 or recess in the surface of the toilet block mitigates these effects and provides improved dissolution of the blocks. A deeper channel may put off the deleterious effects for longer.

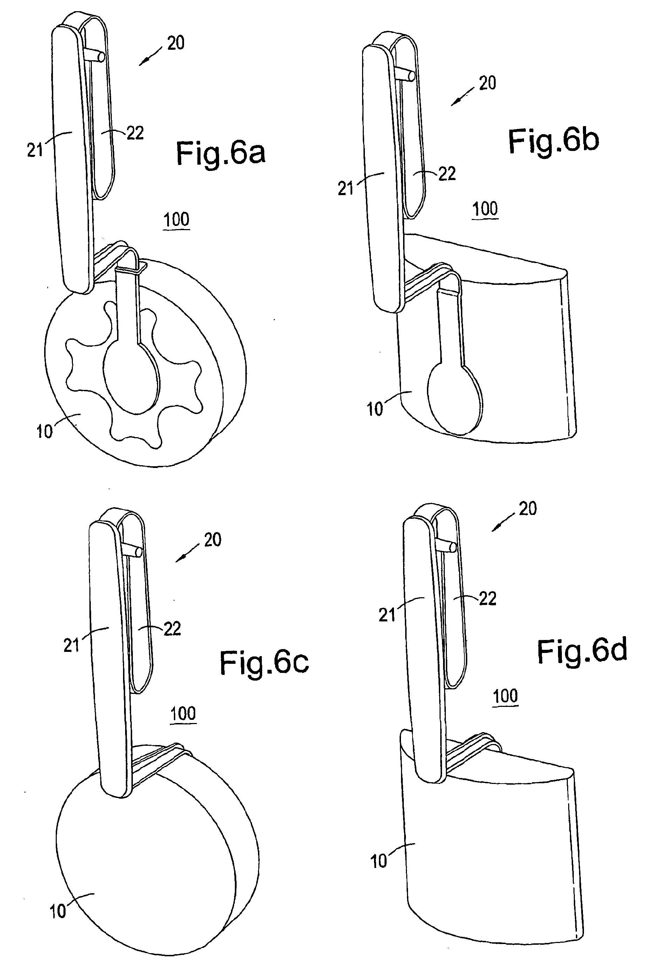

Figures 6a, 6b, 6c, and 6d show variations of the dispensing device. In the examples shown the hook is plain but otherwise is in accordance with the hooks or hangers described above. Elements of the invention described in connection with one embodiment may be combined with other elements as would be evident to the skilled person. In Figure 6 the example have different block shapes that have been produced by an extrusion process only without the need for a subsequent moulding step after cutting the extruded material. Figures 6a and 6b are not covered by claim 6.

Figure 6a shows a co-extruded block 10 where the internal portion is shaped so as to provide a decorative pattern whereas the profile of the block itself is a simple circle. This way a complex and even an irregular pattern can be effected for the block without having an external profile of the block that might be deleterious to the waterflow profile of the device. These properties are critical to the performance of the device and the device factors that affect the water flow profile are predominantly the hanger design - including number of pins and pin position, and the block design. Some block shapes that are complicated can have good water flow profiles that lead to even dissolution in use; however, it is found that regular shapes are more likely to have such good water flow profiles and provide even dissolution. It has been found that the front pinned devices are less affected by other factors and are more likely to have consistent dissolution properties.

Figure 6b shows another simple extruded block, this time with a D shape profile. An advantage of this shape is that the pins of the support portion are inserted into the smooth side wall of the extruded block the cut portions being located at the top and bottom of the block in figure 6b. By contrast the block of figure 6a has the pins inserted directly into the cut face which can lead to less desirable performance in terms of dissolution of the block. The cut face may generally be less aesthetically pleasing since the cut face will show any internal imperfections. Pre-processing of the extruded materials (such as z-blade mixing) may reduce the significance of internal imperfections. Figure 6c and 6d show rear pinned version.

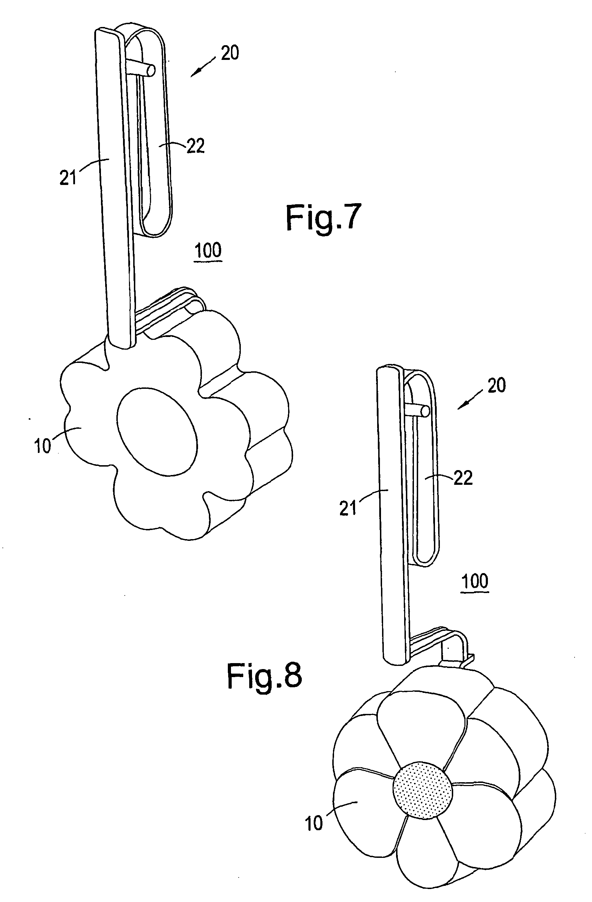

Figure 7 shows a further variation of the block which has been produced by a co-extrusion process to produce a two component block. The inner and outer portions may have different compositions having different colours, fragrances, cleaning effects, active substances, or lifetimes. Co-extruded two part blocks and a process for their manufacture are described in WO-A-2006/070209. The dispensing device of Figure 7 is shown as rear pinned in accordance with the invention but could also be front pinned as previously described. The shape of the block of figure 7 can be produced either by subsequent moulding of the co-extruded cut preform, or the extruded block could have the cross-section shown in figure 7 or any other desired cross-section. The external profile of the block of figure 7 is rather complex having many indents which can lead to uneven dissolution of the block. Since water tends to flow along the indents the block will wear faster at the indents and random factors such as positioning in the bowl are likely to make the block dissolve unevenly even though the shape of the block is even. Simple profiles such as the D-shape and the circle are more likely to have satisfactory performance. However, complex shape can have very good performance and the shapes shown in figures 2a and 2b for example, and figure 8 below have been found, surprisingly to have excellent performance in terms of even dissolution.

Figure 8 shows an example of the moulded product having a so-called 3-D profile, that is the shape of the block is not only defined around the perimeter, but also a relief pattern is also present at least on the front face of the block. The patterned face is produced in a subsequent moulding step as described in relation to figure 1. The block of figure 8 is also a co-extruded block having a central portion having a different composition than the external portion.

Any of the hangers above may be used with any of the described block in any suitable combination.

Whilst embodiments have been described in relation to an extrusion process, it is very common to cast toilet blocks and any of the described hooks could potentially be used also with a cast or tableted blocks.

### EXAMPLES

Compositions for an extruded product were produced in the following ranges:
Anionic Surfactant/ Foamer 20 - 60%
   (e.g.Alpha Olefin Sulphonate (AOS) (80%)
Anionic surfactant/platiciser 3 - 20%
   (e.g. Secondary Alkyl Sulphate (SAS) (92%))

| | |
|---|---|
| Pigments & Dyes | 0 - 0.5% |
| Perfume | 0 - 8% |
| Life extender eg. Neodol 91 or pine oil | 0 - 5% |
| Life shortener, e.g. Mineral Oil | 0 - 5% |
| Filler e.g. Sodium Sulphate | Balance |

Where AOS80% is Nansa LSS480, from Huntsman;
Where SAS92% is Hostapur SAS93G from Clariant;
Neodol 91 is a C9-C11 Primary Alcohol from Shell (hydrophobic oil, used as a life extender). Other hydrophobic oils could be used to extend or shorten life; mineral oil for example, would shorten the life of the block. The use of life extenders (or shortners) is optional and their use depends on, for example, cost and perfume concentration.

The pigments, dyes, perfume, life extenders or shortners, and filler are all optional. Most commercial compostions will include pigment & Dyes, Perfume and filler

Some perfumes are 100% perfumistic materials, whilst other have up to 45% carriers such as di-propylene glycol (DPG). Depending on the oil content of the perfume the addition of further oils, for example life extenders or shorteners are selected to achieve the correct extrusion hardness, life, and fragrance performance, as would be evident to the skilled person.

Formulations equally could use only perfumes, no additional oils - choice is purely to meet a commercial product price target or desired life.

The above compositions were used to form finished products as follows:
i) Mix powders, add oils and mix in plough share or Z-blade mixing system;
ii) Extrude 50mm diameter circular cylinder block;
iii) Cut block to required depth, in this case approximately 13mm (40g);
iv) Fit hook to block, whilst still warm - the hooks used were front pinning hooks having two pins similar to that shown in Figure 4b.

Formulations 1 to were tested to monitor their performance in use. The results for formulations 1 to 12 are set out in Table 1 below.

**Table1**

| | | | | | | | | | | | | |
|---|---|---|---|---|---|---|---|---|---|---|---|---|
| Example AOS(80%) SAS(92%) Neodol 91 Pine Oil Mineral Oil Pigments & Dyes Perfume Orange Sparkle Perfume GreenHythe Perfume KeyLimePie Perfume LavenderSparkle Perfume LimeTimeD Perfume TotallyEve05concmod Sodium Sulphate | 1 | 2 | 3 | 4 | 5 | 6 | 7 | 8 | 9 | 10 | 11 | 12 |
| | | | | | | | | | | | | |
| | 34 | 34 | 34 | 34.1 | 34.1 | 34 | 34 | 34 | 34 | 39 | 29 | 39.2 |
| | 10 | 10 | 10 | 10 | 10 | 10 | 10 | 10 | 10 | 6 | 5 | 4.8 |
| | 0.5 | | | 0.5 | | 1.9 | | 0.5 | 1.9 | | | |
| | | | | | 0.5 | | | | | | | |
| | | | 0.5 | | | | | | | 0.8 | 1.2 | 0.8 |
| | 0.011 | 0.011 | 0.011 | 0.03 | 0.035 | 0.035 | 0.035 | 0.03 | 0.04 | 0.011 | 0.011 | 0.035 |
| | 4.8 | 5.2 | 4.8 | | | | | | | 4.6 | 3 | |
| | | | | 4.6 | 4.6 | | | | | | | |
| | | | | | | 3.3 | 5 | | | | | |
| | | | | | | | | 4.6 | | | | |
| | | | | | | | | | | | | 4.6 |
| | | | | | | | | | 3.3 | | | |
| | Balance | Balance | Balance | Balance | Balance | Balance | Balance | Balance | Balance | Balance | Balance | Balance |
| | | | | | | | | | | | | |
| Av Life - Flushes@12flushes/day | | | | | | | | | | | | |
| | 350 | 380 | 250 | 200 | 240 | 340 | 375 | 395 | 230 | 275 | 210 | 360 |
| | | | | | | | | | | | | |

Each of the compositions had a life of up to 4 weeks when tested in bowl in main water flow @ 12 flushes/ day at between 15 and 20 degrees C. All compositions delivered significant foam levels in use from the first flush, and give equal life in hard and soft water. Each composition dissolved evenly going through a gel phase.

Examples 1 to 9 are formulations that are quite plastic when extruded and do not "cure" immediately. The pins of the hangers do not need to be inserted immediately although that may be convenient for the manufacturing process. These examples should how formulations can easily be modified to vary the in-use life time and to show how different perfumes can also affect life. For example, examples 1 and 4 have similar compositions except for the perfume. Example 1 uses Orange sparkle obtained from IFF and containing no carrier oil, whereas Example 4 uses perfume GreenHythe from Givaudan containing approximately 41% DPG. Examples 2 and 3 demonstrate how adding Mineral oil can shorten the life of the product if required. Examples 4 and 5 show the difference between the use of Neodol and pine oil. Examples 6 and 9 show the difference between the use of the perfume KeyLimePie from Givaudan containing no DPG and the use of perfume TotallyEve05concmod from Givaudan containing no carrier oil. Example 8 can be compared to example 4 showing that the LavendarSparkle perfume obtained from IFF and having 10% DPG leads to a significantly longer life than the use of perfume GreenHythe from Givaudan. Whist the content of carrier oil in the perfume may affect plasticity, the particular perfume used is likely to have more effect on life time.

Examples 10 to 12 are formulations that are slightly plastic when extruded but cure quickly on cooling and it is preferably with such compositions to insert the pins of the hangers while the product is still warm. Example 12 includes perfume LimeTimeD, which can be obtained from Symrise.

It appears from the tests performed by the inventors that the percentage of perfume carrier (DPG or otherwise) did not directly equate to block hardness, cure rate or plasticity and that the actual perfume ingredients likely had a greater effect. In particular, the amount of total oils required depends on the plasticizer/ extrusion aid selected, in this case SAS.

In the present example using AOS and SAS within the ranges described it has been found that total oils content (perfume, life extender, shortening agent, etc.) should be no more than about 5.5 %. However, the amount of perfume that can be added will vary depending on the amounts of surfactant and filler etc.

Since the identity of the perfume material (regardless of a carrier) has effects on the cure rate and block hardness, the formulations will vary for different perfumes in order to maintain similar physical properties between formulations so that processing conditions can be standardised across a range of different fragrance types.

In the Examples of Table 1 a circular cylinder block was used, however, good results have also been obtained with the "D" shaped block of figures 6b and 6d. The advantage of this shape is that the pins are not inserted into the cut face as they are for the circular product. The cut face is generally less smooth and may be prone to uneven dissolution due to surface imperfections. Many other shapes are of course possible and shapes that offer good water flow will dissolve most consistently.

The shape of the block is, therefore, significant importance; other factors include pin positions and the pin arrangement. Addition of the second pin to the hanger aids handling and ongoing location in use, that is, retention of the block on the hanger.

Other compositions that could be used with the dispenser of the invention include those described in WO-A-2007107750 and WO-A-2007107755. The compositions disclosed in these documents are used with a very specific hook design and process involving embedding an enlarged plate portion within the body of the block and compressing the composition onto the plate to ensure proper retention of the block. However, such compositions will work as effectively with the hangers of the dispensers disclosed herein. The hanger design of the present invention, therefore, has the advantage of a simplified process and a reduced material usage since the hangers disclosed herein use approximately 20% less plastic.

The compositions disclosed in WO-A-2007107750 and WO-A-2007107755 use Coconut mono-ethanolomide (CME) or Lauryl mono-ethanolomide (LME) instead of SAS. Use of SAS tends to plasticise, which is beneficial for extrusion and for hook pin retention. The blocks using SAS remain slightly plastic on curing but are not sticky to handle in production or with the consumer. Use of other materials such as eg. Fatty Alcohol Ethoxylate (FA 30EO), or CME, as additives or in place of SAS act as extrusion aids because they melt, but it has been found that the cold blocks are very hard and can embrittle and cause failure - especially around the retention pins. Very careful formulation would be required to use these alternative extrusion aids. Furthermore, the use of SAS leads to less hygroscopic products that are more stable at high temperatures and humidity.

WO-A-2007107750 and WO-A-2007107755 disclose formulations including Linear Alcohol Benzene Sulphonate (LABS) as an anionic surfactant. However, use of LABS tends to increase the propensity of the block to "Mush" in use. The use in the exemplary formulations of SAS instead of LABS leads to less hygroscopic products that are more stable at high temperatures and humidity. Consequently compositions based on AOS/ LABS do not require such stringent packaging requirements where expensive barrier packaging to prevent moisture ingress in transit or storage.

Furthermore, these prior art formulations tend to use fumed silica or other forms of silica to aid their production process and also helps plasticise the composition.

Whist various embodiment of the invention have been described with reference to examples and the figures, variations will suggest themselves to those skilled in the art without departing from the scope of the invention as defined by the appended claims.

## Claims

1. A method of manufacturing a toilet bowl treatment composition dispensing device comprising:
providing a hanger (20) including, an elongate body portion having a first end and a second end, a hook portion (22) the first end for suspending the hanger from a rim of said toilet bowl, and a block supporting portion (23) at the second end, the block supporting portion (23) having at least one finger (25) for receiving a treatment composition block (10) and projecting from a planar portion of the supporting portion (23); the hook portion (22) and the at least one finger (25) project from opposite longitudinal sides of the body portion; and
inserting said at least one finger (25) into a surface of said treatment composition block (10).

2. A method as claimed in claim 1, wherein the treatment composition block (10) is formed by one of, an extrusion process, a tableting process and a hot melt process.

3. A method as claimed in claim 2 wherein the method comprises extruding the treatment composition and cutting into blocks and optionally wherein the extrusion process is a co-extrusion process and the block has at least two distinct portions having different compositions.

4. A method as claimed in any one of claims 1 to 3 wherein before inserting the at least one finger (25) the block (10) is subjected to a shaping process in a mould.

5. A method as claimed in any one of claims 1 to 4 wherein the method includes a step of wrapping the block (10) in a water soluble film.

6. A toilet bowl treatment composition dispensing device, comprising a hanger (20) and a treatment composition block (10), the hanger (20) comprising:
an elongate body portion having a first end and a second end;
a hook portion (22) at the first end for suspending the hanger from a rim of a toilet bowl; and
a block supporting portion (23) at the second end, the block supporting portion (23) having at least one finger (25) projecting from a surface of the supporting portion (23);
wherein the hook portion (22) and the at least one finger (25) project from opposite longitudinal sides of the body portion, and the treatment composition block (10) is retained on the block supporting portion (23) by said at least one finger (25) inserted into a surface of said treatment composition block (10).

7. A dispensing device as claimed in claim 6, wherein there are two fingers (25) on the supporting portion (23).

8. A dispensing device as claimed in claim 6 or 7, wherein the said at least one fingers (25) located on a part of the support portion (23) having an enlarged cross section (24).

9. A dispensing device as claimed in any one of claims 6 to 8, wherein the at least one finger (25) is inserted into a surface of the treatment composition block (10) whereby a surface of the supporting portion (23) from which the at least one finger (25) extends abuts the surface of the treatment composition block (10) into which the finger (25) is inserted and wherein the abutting surfaces are preferably planar.

10. A dispensing device as claimed in any one of claims 6 to 9, wherein the block (10) is cylindrical or wherein the block (10) has two opposing faces that have been subjected to a cutting process and the at least one finger (25) is inserted into a surface of the block other than said opposing faces.

11. A dispensing device as claimed in any one of claims 6 to 10, wherein the at least one finger (25) has at least a portion having a surface that is serrated, barbed or roughened to improve grip and/or wherein the at least one finger has a length that is at least 20% of the depth of the block and no more than 100%, preferably 50 to 95%, more preferably 60 to 85%, most preferably 70 to 80%.

12. A dispensing device as claimed in any one of claims 6 to 11, wherein the treatment composition block (10) is provided with a channel (27) in the surface into which the pins (25) inserted and the channel (27) conforms to the profile of the supporting portion (23) which overlaps said surface of the block.

13. A dispensing device as claimed in any one of claims 6 to 12, wherein the treatment composition block (10) is an extruded block.

14. A dispensing device as claimed in any one of claims 6 to 13, wherein the treatment composition block (10) comprises 20 to 80wt% of at least one surfactant, preferably including an anionic surfactant, and optionally, one or more pigments or dyes, one or more additives acting as life extending or life shortening agents, one or more perfumes, and filler.

15. A dispensing device as claimed in claim 14, wherein the treatment composition block (10) comprises 20 to 60wt% of at least one first surfactant which may be AOS (80%) and 3 to 20% of at least one second surfactant which may be SAS (92%) acting as a plasticizer.

## Patentansprüche

1. Verfahren zur Fertigung einer Abgabevorrichtung für eine Toilettenbecken-Behandlungszusammensetzung, das Folgendes umfasst:
das Bereitstellen eines Anhängers (20), der einen länglichen Körperabschnitt, der ein erstes Ende und ein zweites Ende hat, einen Hakenabschnitt (22) an dem ersten Ende zum Aufhängen des Anhängers an einem Rand des Toilettenbeckens und einen Blocktragabschnitt (23) an dem zweiten Ende einschließt, wobei der Blocktragabschnitt (23) wenigstens einen Finger (25) zum Aufnehmen eines Behandlungszusammensetzungsblocks (10) und der von einem ebenen Abschnitt des Tragabschnitts (23) vorspringt, hat, wobei der Hakenabschnitt (22) und der wenigstens eine Finger (25) von gegenüberliegenden Längsseiten des Körperabschnitts vorspringen, und
das Einsetzen des wenigstens einen Fingers (25) in eine Fläche des Behandlungszusammensetzungsblocks (10).

2. Verfahren nach Anspruch 1, wobei der Behandlungszusammensetzungsblock (10) durch eines von einem Strangpressvorgang, einem Tablettiervorgang und einem Heißschmelzvorgang geformt wird.

3. Verfahren nach Anspruch 2, wobei das Verfahren das Strangpressen der Behandlungszusammensetzung und das Schneiden in Blöcke umfasst und wobei wahlweise der Strangpressvorgang ein gemeinsamer Strangpressvorgang ist und der Block wenigstens zwei verschiedene Abschnitte hat, die unterschiedliche Zusammensetzungen haben.

4. Verfahren nach einem der Ansprüche 1 bis 3, wobei vor dem Einsetzen des wenigstens einen Fingers (25) der Block (10) einem Formungsvorgang in einer Form unterworfen wird.

5. Verfahren nach einem der Ansprüche 1 bis 4, wobei das Verfahren einen Schritt des Einwickelns des Blocks (10) in eine wasserlösliche Folie einschließt.

6. Abgabevorrichtung für eine Toilettenbecken-Behandlungszusammensetzung, die einen Anhänger (20) und einen Behandlungszusammensetzungsblock (10) umfasst, wobei der Anhänger (20) Folgendes umfasst:
einen länglichen Körperabschnitt, der ein erstes Ende und ein zweites Ende hat,
einen Hakenabschnitt (22) an dem ersten Ende zum Aufhängen des Anhängers an einem Rand des Toilettenbeckens und
einen Blocktragabschnitt (23) an dem zweiten Ende, wobei der Blocktragabschnitt (23) wenigstens einen Finger (25), der von einer Fläche des Tragabschnitts (23) vorspringt, hat,
wobei der Hakenabschnitt (22) und der wenigstens eine Finger (25) von gegenüberliegenden Längsseiten des Körperabschnitts vorspringen, und der Behandlungszusammensetzungsblock (10) durch den wenigstens einen Finger (25), der in eine Fläche des Behandlungszusammensetzungsblocks (10) eingesetzt wird, an dem Blocktragabschnitt (23) gehalten wird.

7. Abgabevorrichtung nach Anspruch 6, wobei es zwei Finger (25) an dem Tragabschnitt (23) gibt.

8. Abgabevorrichtung nach Anspruch 6 oder 7, wobei der wenigstens eine Finger (25) an einem Teil des Tragabschnitts (23) angeordnet ist, der einen vergrößerten Querschnitt (24) hat.

9. Abgabevorrichtung nach einem der Ansprüche 6 bis 8, wobei der wenigstens eine Finger (25) in eine Fläche des Behandlungszusammensetzungsblocks (10) eingesetzt wird, wodurch eine Fläche des Tragabschnitts (23), von der sich der wenigstens eine Finger (25) erstreckt, an die Fläche des Behandlungszusammensetzungsblocks (10) anstößt, in die der Finger (25) eingesetzt wird, und wobei die aneinanderstoßenden Flächen im Wesentlichen eben sind.

10. Abgabevorrichtung nach einem der Ansprüche 6 bis 9, wobei der Block (10) zylindrisch ist oder wobei der Block (10) zwei gegenüberliegende Seiten hat, die einem Schneidvorgang unterworfen worden sind, und der wenigstens eine Finger (25) in eine andere Fläche des Blocks als die gegenüberliegenden Seiten eingesetzt wird.

11. Abgabevorrichtung nach einem der Ansprüche 6 bis 10, wobei der wenigstens eine Finger (25) wenigstens einen Abschnitt hat, der eine Fläche hat, die gezahnt, mit Haken versehen oder aufgeraut ist, um den Griff zu verbessern, und/oder wobei der wenigstens eine Finger eine Länge hat, die wenigstens 20 % der Tiefe des Blocks und nicht mehr als 100 %, vorzugsweise 50 bis 95 %, bevorzugterweise 60 bis 85 %, insbesondere 70 bis 80 % beträgt.

12. Abgabevorrichtung nach einem der Ansprüche 6 bis 11, wobei der Behandlungszusammensetzungsblock (10) mit einem Kanal (27) in der Fläche, in welche die Stifte (25) eingesetzt werden, versehen ist und sich der Kanal (27) an das Profil des Tragabschnitts (23) anpasst, das die Fläche des Blocks überlappt.

13. Abgabevorrichtung nach einem der Ansprüche 6 bis 12, wobei der Behandlungszusammensetzungsblock (10) ein stranggepresster Block ist.

14. Abgabevorrichtung nach einem der Ansprüche 6 bis 13, wobei der Behandlungszusammensetzungsblock (10) 20 bis 80 Gew.-% wenigstens eines grenzflächenaktiven Stoffs umfasst, der vorzugsweise einen anionischen grenzflächenaktiven Stoff einschließt, und wahlweise ein oder mehrere Pigmente oder Farbstoffe, ein oder mehrere Zuschlagstoffe, die als die Lebensdauer verlängernde oder die Lebensdauer verkürzende Mittel wirken, ein oder mehrere Duftstoffe und Füllstoff umfasst.

15. Abgabevorrichtung nach Anspruch 14, wobei der Behandlungszusammensetzungsblock (10) 20 bis 60 Gew.-% wenigstens eines ersten grenzflächenaktiven Stoffs, der AOS (80 %) sein kann, und 3 bis 20 Gew.-% wenigstens eines zweiten grenzflächenaktiven Stoffs, der SAS (92 %) sein kann, der als ein Weichmacher wirkt, umfasst.

## Revendications

1. Procédé de fabrication d'un dispositif de distribution de composition de traitement de cuvette de toilette, comprenant les étapes consistant à :
fournir un étrier de suspension (20) comprenant une partie corps allongé présentant une première extrémité et une deuxième extrémité, une partie crochet (22) située au niveau de la première extrémité et permettant de suspendre l'étrier de suspension à un rebord de ladite cuvette de toilette, et une partie support de bloc (23) située au niveau de la deuxième extrémité, la partie support de bloc (23) présentant au moins un doigt (25) permettant d'accueillir un bloc de composition de traitement (10) et faisant saillie à partir d'une partie plane de la partie support (23) ; la partie crochet (22) et le au moins un doigt (25) faisant saillie à partir de côtés longitudinaux opposés de la partie corps, et
insérer au moins un doigt (25) dans une surface dudit bloc de composition de traitement (10).

2. Procédé selon la revendication 1, dans lequel le bloc de composition de traitement (10) est formé grâce à un procédé parmi un procédé d'extrusion, un procédé de pastillage et un procédé de thermofusion.

3. Procédé selon la revendication 2, dans lequel le procédé comprend les étapes consistant à extruder la composition de traitement et découper en blocs et éventuellement dans lequel le procédé d'extrusion est un procédé de co-extrusion et le bloc présente au moins deux parties distinctes présentant différentes compositions.

4. Procédé selon l'une quelconque des revendications 1 à 3, dans lequel, avant insertion du au moins un doigt (25), le bloc (10) est soumis à un procédé de formage dans un moule.

5. Procédé selon l'une quelconque des revendications 1 à 4, dans lequel le procédé comprend une étape consistant à enrouler le bloc (10) dans un film hydrosoluble.

6. Dispositif de distribution de composition de traitement de cuvette de toilette, comprenant un étrier de suspension (20) et un bloc de composition de traitement (10), l'étrier de suspension (20) comprenant :
une partie corps allongé présentant une première extrémité et une deuxième extrémité ;
une partie crochet (22) située au niveau de la première extrémité et permettant de suspendre l'étrier de suspension à un rebord d'une cuvette de toilette ; et
une partie support de bloc (23) située au niveau de la deuxième extrémité, la partie support de bloc (23) présentant au moins un doigt (25) faisant saillie à partir d'une surface de la partie support (23)
dans lequel la partie crochet (22) et le au moins un doigt (25) font saillie à partir de côtés longitudinaux opposés de la partie corps, et le bloc de composition de traitement (10) est retenu sur la partie support de bloc (23) grâce audit au moins un doigt (25) inséré dans une surface dudit bloc de composition de traitement (10).

7. Dispositif de distribution selon la revendication 6, dans lequel il existe deux doigts (25) sur la partie support (23).

8. Dispositif de distribution selon la revendication 6 ou 7, dans lequel ledit au moins un doigt (25) est situé sur une partie de la partie support (23) qui présente une section transversale (24) agrandie.

9. Dispositif de distribution selon l'une quelconque des revendications 6 à 8, dans lequel le au moins un doigt (25) est inséré dans une surface du bloc de composition de traitement (10), grâce à quoi une surface de la partie support (23) à partir de laquelle s'étend le au moins un doigt (25) est attenante à la surface du bloc de composition de traitement (10) dans lequel est inséré le doigt (25), les surfaces attenantes étant de manière préférée planes.

10. Dispositif de distribution selon l'une quelconque des revendications 6 à 9, dans lequel le bloc (10) est cylindrique ou dans lequel le bloc (10) présente deux faces opposées qui ont été soumises à un procédé de découpe et le au moins un doigt (25) est inséré dans une surface du bloc autre que lesdites faces opposées.

11. Dispositif de distribution selon l'une quelconque des revendications 6 à 10, dans lequel le au moins un doigt (25) présente au moins une partie présentant une surface qui est crantée, dentelée ou rendue rugueuse afin d'améliorer la préhension et/ou dans lequel le au moins un doigt présente une longueur qui représente au moins 20 % de la profondeur du bloc et ne dépasse pas 100 %, de manière préférée 50 à 95 %, de manière plus préférée 60 à 85 %, de la manière la plus préférée 70 à 80 % de ladite profondeur.

12. Dispositif de distribution selon l'une quelconque des revendications 6 à 11, dans lequel le bloc de composition de traitement (10) est muni d'un canal (27) dans la surface duquel sont insérées les broches (25) et le canal (27) correspond au profil de la partie support (23) qui surmonte ladite surface du bloc.

13. Dispositif de distribution selon l'une quelconque des revendications 6 à 12, dans lequel le bloc de composition de traitement (10) est un bloc extrudé.

14. Dispositif de distribution selon l'une quelconque des revendications 6 à 13, dans lequel le bloc de composition de traitement (10) comprend 20 à 80 % en poids d'au moins un composé tensioactif, de manière préférée comprenant un composé tensioactif anionique, et éventuellement, un ou plusieurs pigment(s) ou colorant(s), un ou plusieurs additif(s) agissant en tant qu'agents prolongateurs ou réducteurs de vie, un ou plusieurs parfum(s), et une matière de charge.

15. Dispositif de distribution selon la revendication 14, dans lequel le bloc de composition de traitement (10) comprend 20 à 60 % en poids d'au moins un premier composé tensioactif, qui peut être de l'AOS (80 %) et 3 à 20 % d'au moins un deuxième composé tensioactif, qui peut être du SAS (92 %) faisant office de plastifiant.
